## Europäisches Patentamt
## European Patent Office
## Office européen des brevets

(19)

(11) Veröffentlichungsnummer : **0 117 878**
**B1**

(12) # EUROPÄISCHE PATENTSCHRIFT

(45) Veröffentlichungstag der Patentschrift :
09.07.86

(21) Anmeldenummer : 83101982.3

(22) Anmeldetag : 01.03.83

(51) Int. Cl.⁴ : **A 61 K  7/06**

(54) **Haarbehandlungsmittel und Haarbehandlungsverfahren.**

(43) Veröffentlichungstag der Anmeldung :
12.09.84 Patentblatt 84/37

(45) Bekanntmachung des Hinweises auf die Patenterteilung : 09.07.86 Patentblatt 86/28

(84) Benannte Vertragsstaaten :
AT BE CH DE FR GB IT LI LU NL SE

(56) Entgegenhaltungen :
EP-A- 0 001 079
EP-A- 0 058 595
CH-A-   179 254
DE-A- 1 667 930
DE-A- 2 314 019
SOAP COSMETICS CHEMICAL SPECIALTIES, Band
58, Nr. 2, Februar 1980, Seite 115, New York, USA
Janisty, Handbuch der Kosmetik und Richstoffe, Bd
1, 1974 S. 311, S. 211; DRAGOCO-Firmenschrift
"Extrapon Brennessol Spezial"

(73) Patentinhaber : Trensch, Rudolf, Dr.
Kalvarienbergstrasse 28
D-8133 Feldafing/Starnberg (DE)

(72) Erfinder : Trensch, Rudolf, Dr.
Kalvarienbergstrasse 28
D-8133 Feldafing/Starnberg (DE)

(74) Vertreter : Strasse, Joachim, Dipl.-Ing. et al
Strasse und Stoffregen European Patent Attorneys
Zweibrückenstrasse 17
D-8000 München 2 (DE)

**Beschreibung**

Die Erfindung betrifft ein Haarbehandlungsmittel aus einer Lotion und einem Shampoo sowie Verfahren zur Herstellung dieser beiden Komponenten und eine Anwendung des Haarbehandlungsmittels.

Aus der EP-A1-0 058 595 ist eine Haarkur bekannt geworden, welche aus einer Lotion und einer Creme besteht, wobei die Lotion Rosmarin, Thymian, Gewürznelke, Salbei, Lavendel und Nelken- und Kamillekörner enthält und die Creme Bergamottöl, pflanzlichen Alkohol, Öl, Schwefelblüte sowie mindestens 90 % Rückenmark, beispielsweise von Rindern. Da Rindermark den weitaus überwiegenden Anteil der Creme ausmacht, besteht die Befürchtung, daß hierdurch die Kopfhautporen verstopft werden und die übrigen Wirkstoffe an einem Eindringen in die Kopfhaut und damit an einer haarwuchsfördernden Wirkung gehemmt werden.

In der CH-A-179 254 wird ein Haarbehandlungsmittel beschrieben, welches aus 27 verschiedenen Stoffen besteht, die sich grob in tierische Bestandteile wie Rinderrückenmark, Schweinefinne, Fuchsfett, Pferdemähnenfett usw. und pflanzliche Bestandteile wie Rosmarin, Salbei, Stechwinde, Kornblume usw. aufteilen lassen. Zwar sind auch Brennesselwurzel, Brennesselblätter und wilder Meerrettich vorgesehen, der hohe Anteil von Fett und Talg enthaltenden Substanzen läßt es aber als höchst unwahrscheinlich erscheinen, daß Wirksubstanzen in die Kopfhaut eindringen und dort eine Wirkung entfalten können ; hinzu kommt ein Anteil an Tannenharz, welcher eine Verklebung der Haare und eine Verstopfung und Verleimung des Haarbodens bewirken kann, so daß andere Substanzen nicht mehr eindringen können.

Die DE-A-1 667 930 beschreibt ein Haarwuchsmittel aus Brennesselextrakt, Alkohol, Rizinusöl, Eidotter und Zitronensaft sowie ein Haarwasser aus einem Extrakt von — zu gleichen Teilen — Brennessel, Kamille und Pfefferminz, welche mit Alkohol übergossen und zur Bildung des Extraktes mindestens vier Wochen lang sich selbst überlassen werden. Wie neuere Erkenntnisse der amerikanischen Forschung ergeben haben, neigt Eidotter zur Förderung des Verklebens der Haare und hat durch seinen Gehalt an Lecithin einen negativen Effekt auf Erhaltung und Neuwuchs der Haare.

Aus der DE-A-2 314 019 ist die Verwendung von Organextrakten aus auf spezielle Weise gewonnenen Haarfollikeln bekannt, denen Thymusextrakte zugesetzt werden können. In Soap Cosmetics Chemical Specialties, Vol. 58 (1982), Februar, No. 2, New York, USA wird ein Shampoo beschrieben, welches neben mehreren anderen Bestandteilen TEA-Laurylsulfat, Kokosnuß-Diethanolamid und Zitronensäure enthält ; ein anderes bekanntes Antifett-Shampoo enthält neben zahlreichen anderen Substanzen Kokosfettdimethylaminoxyd. Dieser Stoff hat hauptsächlich eine nachfettende und in geringerem Maße reinigende Wirkung.

Trotz dieser bekannten Mittel sind Haarausfall und Haarschwund eine zunehmende Erscheinung. Während bislang der Haarausfall oder die Alopezie bis zur Glatze als eine typische Männererscheinung angesehen wurde, hingegen den Frauen nur eine mehr oder minder ausgeprägte Lichtung der Haare zugeschrieben wurde (FEY : « Wörterbuch der Kosmetik », Stuttgart, 1974, Seite 147) läßt sich in jüngster Zeit feststellen, daß, eventuell auch durch Einnahme von Empfängnisverhütungsmitteln, auch bei Frauen die vollständige oder nahezu vollständige androgenetische Alopezie häufiger als früher auftritt. Weder die Haarkosmetik noch die Medizin verfügen hiergegen bislang über wirksame Mittel (FEY a. a. O).

Im Gegensatz zum Nagel an Händen und Füßen des Menschen, der einmal gebildet wird und lebenslänglich wächst, haben die menschlichen Haare eine begrenzte Lebensdauer. Der daraus resultierende natürliche Haarausfall ist ein normaler Vorgang, der als Haarwechsel bezeichnet wird. Der tägliche Verlust in der Größenordnung von 50 Haaren ist bedeutungslos. Vielfach wird aus verschiedenen Gründen diese natürliche Anzahl überschritten und ein unnatürlicher Haarausfall setzt ein, der verschiedene Gründe haben kann und keinesfalls als krankhafte Erscheinung angesehen werden muß. Die Anwendung von Maßnahmen gegen unnatürlich hohen Haarausfall ist deshalb dem Gebiet der Kosmetik zuzuordnen.

Die Erfindung steht daher unter der Aufgabe ein neues Haarbehandlungsmittel zur Verfügung zu stellen, welches eine nachweisbare Wirkung auf den Haarwuchs ausübt.

Eine weitere Aufgabe besteht in der Angabe eines Verfahrens zur Herstellung der Lotion bzw. des Shampoos sowie einer Anwendung des Haarbehandlungsmittels.

Gemäß der Erfindung wird ein Haarbehandlungsmittel aus einer Lotion und einem Shampoo bereitgestellt, bei welchem die Lotion eine Lösung oder Dispersion von 28 g Meerrettichsaft, 5fach konzentriert, etwa 63° Brix, 34 g Brennesselextrakt in Glykolen aufbereitet (« Extrapon »), 2 g Vitamin B6, 2 g Vitamin E wasserlöslich (1 ml = 500 mg DL-a-Tocopherolacetat), 2 g d-Panthenol, 10 g Cremophor® (Alkohol-Wasserlösliches Rizinusöl) und 32 g Diisopropyladipat in 408 g Isopropylalkohol aufgefüllt auf 1 000 g mit destilliertem Wasser darstellt. Das Shampoo besteht aus 250 g Alkyldiglykoläthersulfatnatrium, 541 g Triäthanolaminlaurylsulfat, 150 g Kokosfettdimethylaminoxyd, 8 g Meerrettichsaft, 5fach konzentriert, etwa 63° Brix, 3 g Cremophor® (Alkohol-Wasser-lösliches Rizinusöl), 9 g spanischem Rosmarinöl, 8 g Isopropylalkohol, 10 g Zitronensäure, 10 g Natriumchlorid und 20 g destilliertem Wasser. Der Meerrettichsaft in Lotion und Shampoo kann jeweils durch die entsprechende Menge Allylsenföl ersetzt werden. Bei einer weiteren Ausführungsform der Erfindung ist vorgesehen, daß die Lotion 6 g sprühgetrockneten Thymusdrüsenextrakt enthält.

Bei dem Verfahren zur Herstellung der Lotion ist vorgesehen, daß 28 g Meerrettichsaft, 5fach konzentriert, etwa 63° Brix, 35 g Brennesselextrakt in Glykolen aufbereitet (« Extrapon »), 2 g Vitamin B6, 2 g Vitamin E wasserlöslich (1 ml = 500 mg DL-a-Tocopherolacetat), 2 g d-Panthenol, 10 g Cremophor® (Alkohol-Wasserlösliches Rizinusöl), 32 g Diisopropyladipat in 408 g Isopropylalkohol und destilliertes Wasser ad 1 000 g vermischt werden, und die entstehende Mischung nach etwa vier Wochen filtriert wird. Hierbei können die 28 g Meerrettichsaft wie erwähnt durch 28 g Allylsenföl ersetzt werden. Es ist weiterhin vorgesehen, vor der Zugabe von destilliertem Wasser ad 1 000 g 6 g sprühgetrockneten Thymusdrüsenextrakt zuzugeben.

Bei dem Verfahren zur Herstellung des Shampoos ist vorgesehen, daß 250 g Alkyldiglykoläthersulfatnatrium, 541 g Triäthanolaminlaurylsulfat, 150 g Kokosfettdimethylaminoxyd, 8 g Meerrettichsaft, 5fach konzentriert, etwa 63° Brix, 3 g Cremophor® (Alkohol-Wasserlösliches Rizinusöl), 9 g spanisches Rosmarinöl, 8 g Isopropylalkohol, 10 g Zitronensäure, 10 Natriumchlorid und 20 g destilliertes Wasser vermischt werden. Können die genannten 8 g Meerrettichsaft durch 8 g Allylsenföl ersetzt werden.

Bezüglich der Anwendung der Haarbehandlung wird vorgeschlagen, daß die Kopfhaut täglich früh und abends mit der Lotion eingerieben wird und die Haare und die Kopfhaut alle zwei bis drei Tage mit dem Shampoo gewaschen werden.

Von den benutzten Wirkstoffen werden einige bereits für sich oder in anderer Kombination benutzt, ohne die bislang angestrebte Wirkung zu erzielen. So ist es insbesondere bekannt, Brennesselextrakt sowohl für Haarwasser als auch für Shampoo zu verwenden (DE-AI-1 667 930 von Karl Waldmann, offengelegt am 20.04.1972 ; Dr. Hirsch : « Neue Erkenntnisse der Haarforschung », Schweiz, 1955, Seite 42 ; Curt Humins : « Pharmazeutisches Wörterbuch », Berlin, New York, 1975, 5. Auflage, Seite 709 unter Stichwort « Urtica-Arten » ; H. Fey : « Wörterbuch der Kosmetik », Stuttgart, 1974, Seite 159, unten, im Stichwort « Haarwasser » ; Karl Fries : « Kann Haarausfall und Haarschwund aufgehalten werden ? », Ottobrunn bei München, 1952, Seite 16 unten). Ein für Haarwasserzusätze aufbereiteter Brennesselextrakt als eine in Wasser lösliche braune Flüssigkeit mit einem pH-Wert von 6-7 ist unter der Bezeichnung Extrapon, Firma DRAGOCO Gerberding & Co. GmbH, bekannt. In der vorliegenden Erfindung wird die bekannte Wirkung dieses Bestandteils zusätzlich und in Kombination mit den anderen Wirkstoffen mitbenutzt.

Wenn auch der Wirkungsmechanismus des neuen Haarbehandlungsmittels in seiner Gesamtheit trotz eingehender Versuche noch nicht vollständig geklärt ist, so lassen sich doch Anhaltspunkte für die Wirkung einiger Komponenten geben. Die Wirkung der Einreibung beruht auf dem Zusammenwirken von Meerrettichsaft 5fach konzentriert, durch Allylsenföl ersetzbar, und Brennesselextrakt in Glykolen aufbereitet. In erster Linie wird hierdurch der Haarausfall verhindert.

Der fakultative Zusatz von Thymusdrüsenextrakt stärkt die Wirkung gegen Haarausfall und führt zusätzlich zu einem Wachstum neuer Haare, sofern die Haarfollikel noch vorhanden sind. Die neuwachsenden Haare sind stark und in fast allen Fällen wieder neu pigmentiert, wodurch sich auch ein volleres Aussehen der Haare ergibt.

Diisopropyladipat zusammen mit dem d-Panthenol und Cremophor® fördert den Eingang hauptsächlich des Thymusdrüsenextraktes in die Kopfhaut bis an die Haarwurzel. Weiterhin bewirken sie eine bessere Kämmbarkeit des Haares und Diisopropyladipat verhindert weitgehend ein etwaiges Kleben.

Brennesselextrakt in Glykolen bewirkt eine deutliche Reduktion der Talgabsonderung durch die Talgdrüsen.

Die günstige Wirkung von Vitamin B6, des dl-a-Tocopherolacetates und des Cremophor® auf die Haarwurzeln ist bekannt. Es wurde festgestellt, daß diese Zusätze die Wirkung des Meerrettichkonzentrates, des Brennesselextraktes und des Thymusdrüsenextraktes wesentlich unterstützen und verstärken. Schließlich dient die Zitronensäure dazu, einen pH-Wert von etwa pH 5 zu schaffen.

Beim Shampoo sind die Schaumbildung und gleichzeitige Reinigungswirkung des Alkyl-diglykoläthersulfat-Natrium wichtig, während das Triäthanolaminlaurylsulfat außer seines reinigenden Effektes eine beachtliche Reduktion der freien Fettsäuren und Zunahme der Triglyzeride auf und in der Kopfhaut bewirkt. Haarausfälle werden auch dadurch reduziert. Die Wirkung wird noch verstärkt durch Anwesenheit des Meerrettichkonzentrates, des Cremophor® und des spanischen Rosmarinöls, das zusätzlich eine Parfümwirkung entfaltet.

Kokosfett-dimethylaminoxyd bewirkt eine leichte Nachfettung des Haares, entspannt das Haar und macht es leicht kämmbar.

Es ist wichtig, daß Lotion und Shampoo zusammen benutzt werden. Durch die Substanzen beider Produkte wird der Haarausfall geregelt, bzw. vermieden oder neuer Haarwuchs oder die Stärkung von geschwächten Haaren angeregt und gefördert, sowie neue Pigmentierung eingeleitet. Das Einreiben der Haare mit der Lotion zweimal täglich, am besten früh und abends, verbunden mit einer Haarwäsche mit dem Shampoo alle 2 bis 3 Tage hat sich als das Behandlungsverfahren erwiesen, bei dem die besten Ergebnisse erzielt wurden.

Das Zusammenwirken von Lotion und Shampoo im erfindungsgemäßen Haarbehandlungsmittel und dessen Wirksamkeit wurde klinisch getestet und kann als bedeutender Fortschritt bei der Bekämpfung von Haarausfall und zur Förderung des Neuwachstums, wofür es bisher kaum erfolgreiche Präparate gab, bezeichnet werden.

Besonders beachtenswert sind die Versuche und Resultate durch Haarstatus (Trichogramm = Untersuchung der Haarwurzel), wie sie von der Universität in München unter Berechnung durch das Institut für Medizinische Informatik und Systemforschung nachgewiesen wurde, wonach

0 117 878

die Umwandlung von telogenen Haaren (Ausfallhaaren) zu anagenen Haaren (Wachstumshaaren) bei 8 % bis 30 % lag und immer der Normalstand 80 % anagener Haare erzielt wurde. In allen bisher behandelten 1 800 Fällen wurde auch nachgewiesen, daß durch die vorstehende Haarbehandlung in keinem einzigen Fall negative Nebenwirkungen (Schäden an der Kopfhaut oder den Haaren) in irgendeiner Form aufgetreten sind.

## Beispiel 1

Mit einer Lotion gemäß Anspruch 3 wurde die Kopfhaut zweimal täglich, morgens und abends, eingerieben und jeden 2. oder 3. Tag durch eine Kopfwäsche mit einem Shampoo gemäß Anspruch 3 ergänzt. Die zugemessene Shampoomenge bildet, wenn auf der befeuchteten Kopfhaut gleichmäßig verteilt, reichhaltigen Schaum, der 1 bis 3 Minuten auf der Kopfhaut verbleibt, dann wurde nach reichlicher Spülung mit warmen fließendem Wasser getrocknet. Danach erfolgte eine wiederholte Einreibung mit der Lotion. Die Kopfwäsche wurde jeweils nur einmal mit dem Shampoo durchgeführt.

Eine jeweilige Behandlungsdauer erstreckte sich, dem Ausmaß des Haarausfalles entsprechend, 4 bis 6, bzw. 8 Wochen. Zu Kurbeginn wurde dem klinischen Zustand der Kopfhaut entsprechend in jedem einzelnen Fall zusammen mit anamnestischen Angaben ein detaillierter Status aufgestellt. Dieser enthielt das Ausmaß des Dünnerwerdens des Haarwuchses oder des Haarausfalles, die Anzahl der von Morgen bis Abend ausgefallenen Haare, den Grad des Schuppenbefalls und der Haarfettung ferner entzündliche Symptome, juckende Beschwerden. Der tägliche Haarverlust wurde von den Versuchspersonen bei Kurbeginn und während der Kur bei wöchentlicher Kontrolle selbst gezählt. Während der Kur erschienen die Versuchspersonen zweiwöchentlich zu Kontrolluntersuchungen und nach Beendigung der Kur monatlich, ferner nach sechs Monaten zur Registrierung von Haarregeneration und eventuellen Rezidiven. Während dieser Zeitperiode erhielten die untersuchten Personen keine sonstige ähnliche Behandlung.

Eine kurmäßige Behandlung mit der Lotion und dem Shampoo erhielten insgesamt 128 Versuchspersonen : 68 Männer und 60 Frauen, im Alter von 17 bis 60 Jahren. Davon hatten 88 Versuchspersonen, 66 Männer und 22 Frauen, seborrhoischen Haarausfall, mit und ohne Glatze ; in 20 Fällen wurde der verstärkte Haarausfall durch die Einnahme von Kontrazeptiva-Tabletten verursacht ; 10 Versuchspersonen litten unter klimakterischem Haarausfall, bei 10 Versuchspersonen standen andere vermutliche Gründe, Alopecia areata, Pseudopelade Brocq, Gravidität hinter dem diffusen Haarausfall.

Die im Verlauf der Behandlung aufgetretenen objektiven und subjektiven Nebeneffekte wurden durch Beobachtungen der jeweiligen Versuchspersonen festgestellt. Irritative und sensibilisierende Effekte des neuen Präparates wurden mit der Lotion ohne Verdünnung untersucht : 20 Personen wurden mit einer einmaligen 48-stündigen Okklusionsauftragung auf der Rückenhaut und auf der gleichen Oberfläche auf ähnliche Weise mit mehrmaligen 4 bis 5mal ausgeführten Auftragungen getestet. Die Untersuchung der Einwirkung von Licht (fototoxische Wirkung) hinsichtlich des Inhaltes der pflanzlichen Extraktwirkstoffe wurde an 10 Personen erprobt : nach 24stündiger okklusiver Auftragung mit UV-Strahlendosis Exposition bis unterhalb der Erythemaschwelle. Die Hautreaktionen wurde 48 bis 72 Stunden danach ohne Befund ausgewertet.

Objektive Untersuchungen der Hemmung des Haarausfalles erfolgte unter ähnlichen Vorsichtsmaßnahmen in regelmäßigen Zeitabschnitten, wöchentlich immer an den Tagen vor der Kopfwäsche, und bestanden aus dem Zählen der täglich zwischen morgens 7 Uhr bis abends 7 Uhr ausgefallenen Kopfhaare.

Dies wurde mit dem der Jahreszeit entsprechenden normalen Haarausfall verglichen und das Resultat wurde im Vergleich mit der Anzahl der ursprünglich ausgefallenen Kopfhaare ausgewertet.

Abhängig von der Jahreszeit wird ein tägliches Ausfallen von 20 bis 30 Kopfhaaren als normal betrachtet. Bei der Auswertung der Behandlung oder des Regenerationsgrades wurde mit « ++ » bezeichnet, wenn der Haarausfall gänzlich zum Stillstand kam oder sich normalisierte. Gebessert und mit « + » bezeichnet werden in der Tabelle 1 die Fälle, bei denen im Vergleich zu dem Zustand vor der Behandlung das Ausmaß des Haarausfalles sich wesentlich verringerte : zu einem Drittel oder zumindest zur Hälfte des Anfangswertes.

Der Typ des Haarausfalles, die diagnostische Aufteilung und die Resultate der gemeinsamen Wirkung der Lotion und des Shampoos wird in der Tabelle 1 gezeigt.

Daraus ist zu entnehmen, daß von den 128 Fällen, denen eine Behandlung zuteil wurde, bei 80 Versuchspersonen also in 62,5 % der Fälle der Haarausfall gänzlich zum Stillstand kam, oder sich normalisierte. In 40 Fällen, d. h. in 31,3 % wurde eine wesentliche Besserung erreicht. In insgesamt 8 Fällen war die Behandlung erfolglos, das sind 6,3 %, da der Haarausfall in erhöhter Intensität auch nach einer Kur von 6 bis 8 Wochen weiterhin fortbestand. In diesen Fällen stellte sich später heraus, daß im Hintergrund des Haarausfalles bei 5 Versuchspersonen Alopecia areata und bei 3 Versuchspersonen eine Pseudopelade Brocq Progression als Ursache der Erfolglosigkeit stand. In zwei Fällen verschwand der wegen Schwangerschaft aufgetretene Haarausfall binnen 4 Wochen. Ebenfalls rasch, nämlich binnen maximal 4 bis 6 Wochen, konnte der durch verschiedene Kontrazeptiva verursachte Haarausfall in den Griff bekommen werden.

In 18 Fällen verschwand der Haarausfall der Versuchspersonen, in einem Fall verringerte er sich auf ein Minimum. Auch bei Defluvium klimacterica war die Behandlung erfolgreich : In allen 10 Fällen normalisierte sich allmählich der Haarausfall binnen 4 bis 6 Wochen.

4

Tabelle 1

| Diagnose | Zahl der Fälle | Geschlecht | | Behandlungszeit/Wochen | | | Resultat | | |
|---|---|---|---|---|---|---|---|---|---|
| | | m | w | 4 | 6 | 8 | ++ | + | ∅ |
| Defluvium /et alopecia/ seborrhoica | 88 | 68 | 22 | 18 | 40 | 30 | 50 | 38 | - |
| Defluvium klimacterica | 10 | - | 10 | 4 | 6 | - | 10 | - | - |
| Defluvium anticontraceptiva | 20 | - | 20 | 10 | 10 | - | 18 | 2 | - |
| Defluvium gravidarum | 2 | - | 2 | 2 | - | - | 2 | - | - |
| Defluvium obs.ad alopecia areata | 5 | 1 | 4 | - | 1 | 4 | - | - | 5 |
| Defluvium obs.ad pseudopelade Brocq | 3 | 1 | 2 | - | 3 | - | - | - | 3 |
| Insgesamt: | 128 | 68 | 60 | 34 | 60 | 34 | 80 | 40 | 8 |

0 117 878

Bei den 88 wegen Haarausfall seborrhoischen Typs in Behandlung genommenen Versuchspersonen, nämlich 66 Männer und 22 Frauen, kam in 50 Fällen, das sind 56,8 %, der Haarausfall zum Stillstand, in 38 Fällen, 43,2 %, verminderte sich der tägliche Haarverlust hochgradig. Ohne Besserung ist kein behandelter Fall vorgekommen, aber zur Erreichung des erwünschten Resultates war bei 30 Versuchspersonen eine Behandlung von 8 Wochen notwendig.

Die Dauer der Behandlung wurde meistens von der Stärke des Haarausfalles bestimmt, die auch bei den Rezidiven nach den Kuren eine Rolle spielte. Diese Zusammenhänge werden in der folgenden Tabelle 2 aufgezeigt.

Wenn der tägliche Haarverlust 100 Kopfhaare nicht übersteigt, das waren 32 Fälle, wurde der Haarausfall mit « schwach » qualifiziert, zwischen 100 bis 200 Kopfhaaren, das waren 61 Fälle, mit « mittel », bei 200 bis 300 Kopfhaaren, das waren 28 Fälle, mit « stark » und bei über 300 Kopfhaaren, das waren 7 Fälle, mit « sehr stark ». Bei der Mehrzahl der untersuchten Patienten, nämlich bei 93 von 128, bei denen der Haarverlust täglich 200 Kopfhaare nicht erreichte, wurden mit einer 4- bis 6wöchigen Kur sehr gute Resultate erzielt. Darunter war auch ein Fall, bei dem der Haarausfall schon in der dritten Woche zum Stillstand kam. Es gab sogar auch solche Fälle, bei denen sich neben dem Verschwinden des Haarausfalles auch eine Haarregeneration zeigte und nur aus diesem Grunde wurde die Behandlung auch noch nach der 6. Woche fortgesetzt.

Tabelle 2

| vorheriger Kopfhaar-verlust pro Tag | Zahl der Fälle | Geschlecht | | Behandlungszeit/Woche | | | Resultat | | | Rezidiven/Mo | |
|---|---|---|---|---|---|---|---|---|---|---|---|
| | | m | w | 4 | 6 | 8 | ++ | + | Ø | 3 | 6 |
| unter 100 "schwach" | 32 | 12 | 20 | 20 | 12 | - | 30 | 2 | - | - | - |
| 100 – 200 "mittel" | 61 | 41 | 20 | 14 | 37 | 10 | 39 | 20 | 2 | 4 | 10 |
| 200 –300 "stark" | 28 | 11 | 17 | - | 11 | 17 | 11 | 15 | 2 | 4 | 10 |
| über 300 "sehr stark" | 7 | 4 | 3 | - | - | 7 | - | 3 | 4 | 2 | - |
| Insgesamt: | 128 | 68 | 60 | 34 | 60 | 34 | 80 | 40 | 8 | 10 | 20 |

0 117 878

Aus der Tabelle 2 ist auch ersichtlich, daß zur Beseitigung eines täglichen Haarverlustes von über 200 Kopfhaaren in einem Fall die Kur von 4 Wochen nicht ausreichend war. In der Mehrzahl der Fälle erfolgte eine Behandlung von 8 Wochen, z. B. bei extrem hochgradigem seborrhoischen Haarausfall.

Bei den erfolgreich behandelten und zur Kontrolle erschienen Versuchspersonen wurden insgesamt in 30 Fällen Rezidive beobachtet. In 10 Fällen traten diese innerhalb von 3 Monaten nach Beendigung der Kur, in 20 Fällen innerhalb von 6 Monaten auf. Der wiederholt auftretende Haarausfall bei den Patienten war aber immer von geringerem Ausmaß im Vergleich zu dem Haarverlust vor der Behandlung und er konnte auch in kürzester Zeit, maximum 3 bis 4 Wochen, wieder zum Stillstand gebracht werden.

Auf einen Zusammenhang zwischen Intensität des Haarausfalles und der Rezidivbereitschaft weist auch hin, daß bei starkem Haarausfall in 48 %, bei mittlerem Haarausfall nur in 23 % und bei schwachem Haarausfall in keinem Fall Rezidive aufgetreten sind.

Außer dem Verschwinden des Haarausfalles konnte auch bei den untersuchten seborrhoischen Fällen eine sonstige objektive Besserung beobachtet werden. Die mit juckenden Beschwerden einhergehende schuppige Schälung verschwand in jedem Fall, die erhöhte Fettigkeit verringerte sich fallweise zum Normalen. Mit der Veränderung der Quantität und Qualität der Kopfhaare zeigte sich auch in gewissen Fällen eine Haarregeneration. Bei 9 Versuchspersonen wurde hauptsächlich im Anfangsstadium des seborrhoischen Haarausfalles beobachtet, daß auf Einwirkung der Lotion und der Waschkur auf den mit spärlichem Kopfhaar oder Lanugokopfhaar bedeckten Stellen, ferner auf den Grenzgebieten des erhöhten Haaransatzes sich neben der Stärkung der bereits vorhandenen Kopfhaare auch wenige neue kleine Kopfhaare bildeten. Gleichzeitig mit dem Längenwuchs und der Verstärkung der einzelnen Kopfhaare konnte in einigen Fällen eine schwache Pigmentation der Lanugohaare beobachtet werden.

Gleichzeitig mit den Untersuchungen gemäß Beispiel 1 wurden andere Untersuchungen gemäß

Beispiel 2

vorgenommen, denen ebenfalls ein Mittel nach Anspruch 3 zugrunde gelegt wurde.

An den Untersuchungen nahmen 48 Versuchspersonen, 9 Frauen und 39 Männer, teil, die androgenetische Alopezie aufwiesen. Die Behandlung erstreckte sich, soweit möglich, über 12 Wochen. Während dieser Zeit kamen keine anderen äußerlichen oder innerlichen Haarbehandlungsmittel zur Anwendung. Die Anwendung erfolgt gemäß Anspruch 1 folgendermaßen : Zweimal täglich, morgens und abends, wurde die Kopfhaut gut mit der Lotion befeuchtet und leicht einmassiert. Alle 2 bis 4 Tage erfolgte eine Kopfwäsche mit dem Haarshampoo, welches leicht in die Kopfhaut einmassiert und nach einigen Minuten mit lauwarmem Wasser gründlich ausgewaschen wurde.

Vor, nach 4-wöchiger und nach 12-wöchiger Behandlung wurden zum einen subjektive Angaben der Versuchspersonen über Schmerzen und Juckreiz im Bereich des Haarbodens sowie über die Stärke des Effluviums geprüft. Zum anderen wurden vor, unter und nach Therapie objektive Symptome wie Seborrhoe, Kopfschuppen und Haarlichtung in ihrem Ausmaß immer durch denselben Untersucher beurteilt. Geachtet wurde auch auf eine eventuell auftretende verstärkte Pigmentierung der Haare. Ferner wurde zu Beginn der Behandlung, nach 4-wöchiger und nach 12-wöchiger Behandlung, das Haarwurzelmuster für die frontale, parietale und occipitale Region des Capillitiums bestimmt.

In Abwandlung der Technik nach van Scott (Scott, E. J. van : Reinertson, R.P. : Steinmuller, R. : The growing hair roots of the Human scalp and morphology changes therein following amethopterin therapie. J. Invest. Derm. 29, 197-204, 157) wird nach Braun-Falco (1. Braun-Falco, O. : Dynamik des normalen und pathologischen Haarwachstums. Arch. klin. exp. Derm. 227, 419-452, 1966, 2. Braun-Falco, O. : Fischer, Ch. : Über den Einfluß des Haarwachsens auf das Haarwurzelmuster. Arch. klin. exp. Derm. 226, 136-143, 1966, 3. Braun-Falco, O. : Rassner, B. : Über den Einfluß der Epilationstechnik auf normale und pathologische Haarwurzelmuster. Arch. klin. exp. Derm. 223, 501-508, 1965, 4. Braun-Falco, O. : Zaun, H. : Zum Wesen der chronischen diffusen Alopezie bei Frauen. Arch. klin. exp. Derm. 215, 165-180, 1962) ein Büschel von 50 bis 70 Haaren synchron mit raschem Zug und parallel zur Verlaufsrichtung der Haare epiliert. Dazu wird eine spezielle Epilationsklemme verwendet. Um Schrumpfungsvorgänge an den Haarwurzeln zu vermeiden, werden die Haare unmittelbar nach der Epilation in eine Petrischale eingebracht, die einen angefeuchteten Wattebausch enthält und so als feuchte Kammer dient. Die Aufarbeitung des gewonnenen Materials erfolgt baldmöglichst. Der proximale, die Haarwurzeln tragende Anteil des Haarschaftes wird zwischen zwei Objektträgern in physiologischer Kochsalzlösung eingebettet, der distale Anteil verworfen. Die einzelnen Haarwurzeln werden mit einem Binokularmikroskop bei 31facher Vergrößerung in ihre verschiedenen Formen differenziert. Nach Registrierung der absoluten Zahlen erfolgt die Errechnung des prozentualen Anteils der aufgetretenen Haarwurzelformen, des Trichorhizogramms, bzw. Haarwurzelstatus. Bezüglich der unterschiedlichen Merkmale der einzelnen Haarwurzelformen sei auf die oben zitierte Literatur verwiesen. In der vorliegenden Studie wurden folgende Haarwurzelformen registriert : Anagen-, Telogen-, dystrophische und pseudodystrophische Haare.

Bei der Durchführung des Versuches wurde Wert auf Einhaltung des geforderten Zeitraumes von 7 Tagen zwischen letzter Haarwäsche und Epilation der Haare gelegt. Dies war allerdings nicht in allen Fällen gewährleistet. In der Vorstellung, daß die Zunahme pseudodystrophischer Haare entweder Folge der letzten Haarwäsche oder zu langsamer Epilation ist, wurde bei sämtlichen Untersuchungen der Anteil pseudodystrophischer Haarwurzeln als anagene Haarwurzeln interpretiert.

Während der Behandlung mit dem Mittel gemäß der Erfindung sollte sich das Haarwachstum bessern, was im Haarwurzelmuster seinen Ausdruck in einer Zunahme der anagenen Haare finden würde. Gleichzeitig würde logischerweise der Anteil der telogenen Haare sinken, da im vorliegenden Fallbereich der Anteil der dystrophischen Haare ohnehin jeweils nahe 0 % liegt. Jeweils zu den drei Bereichen der behaarten Kopfhaut wird Beobachtungsmaterial gewonnen : frontal, parietal und occipital. Es wurde lediglich eine Behandlungsgruppe rekrutiert, so daß der Hinweis auf Wirksamkeit nur über den historischen Vergleich (vorher gegen nachher mit 3 Meßpunkten ; Beginn, 1 Monat, 3 Monate), sowie Kenntnis über die Normalsituation (Anteil der anagenen Haare um 80 %) gesucht wurde.

Insgesamt wurden 46 auswertbare Fälle zur statistischen Bearbeitung übergeben. Davon lagen allerdings lediglich zu 16 Fällen über alle 3 Zeitpunkte Angaben vor und zu lediglich 25 Fällen zu den beiden ersten Zeitpunkten.

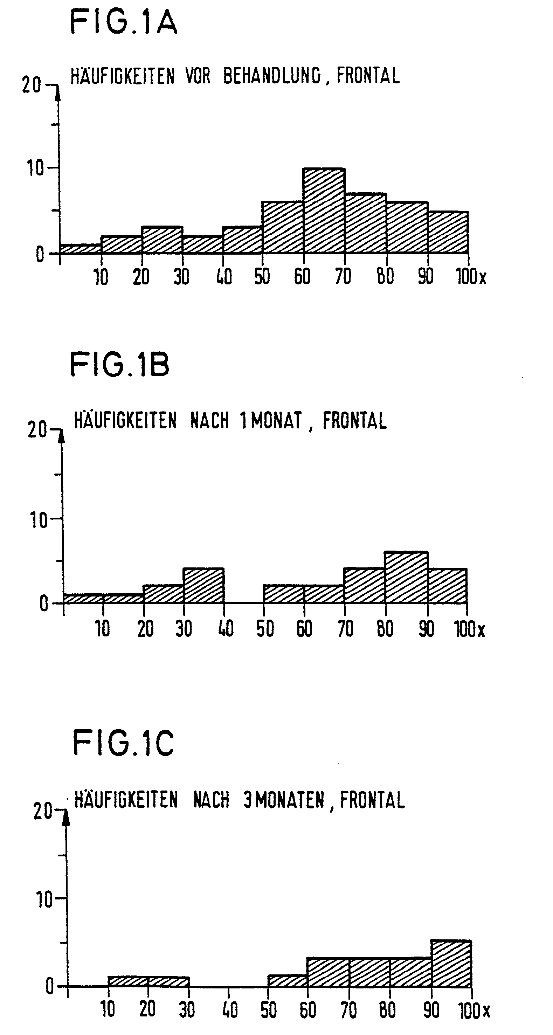

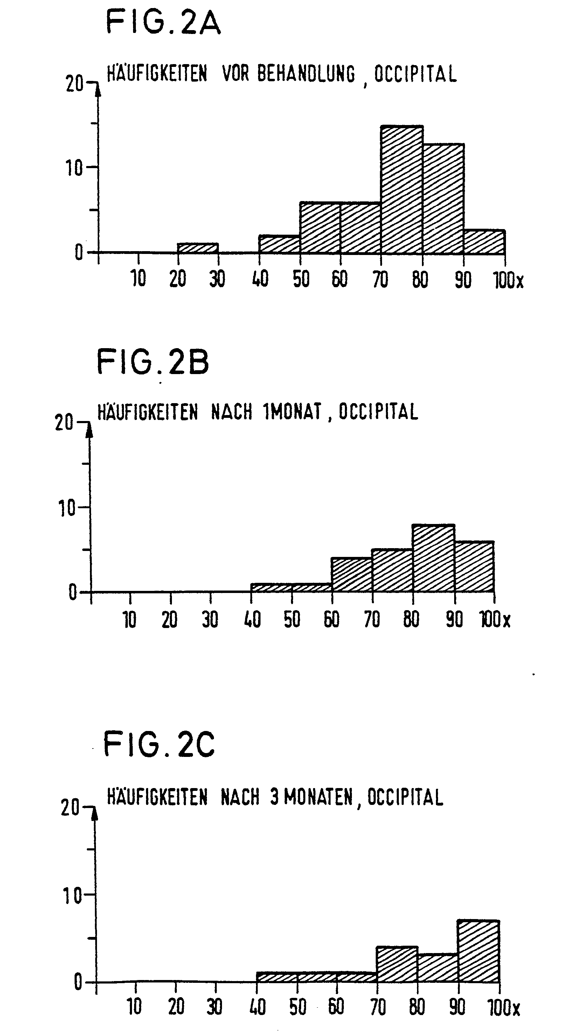

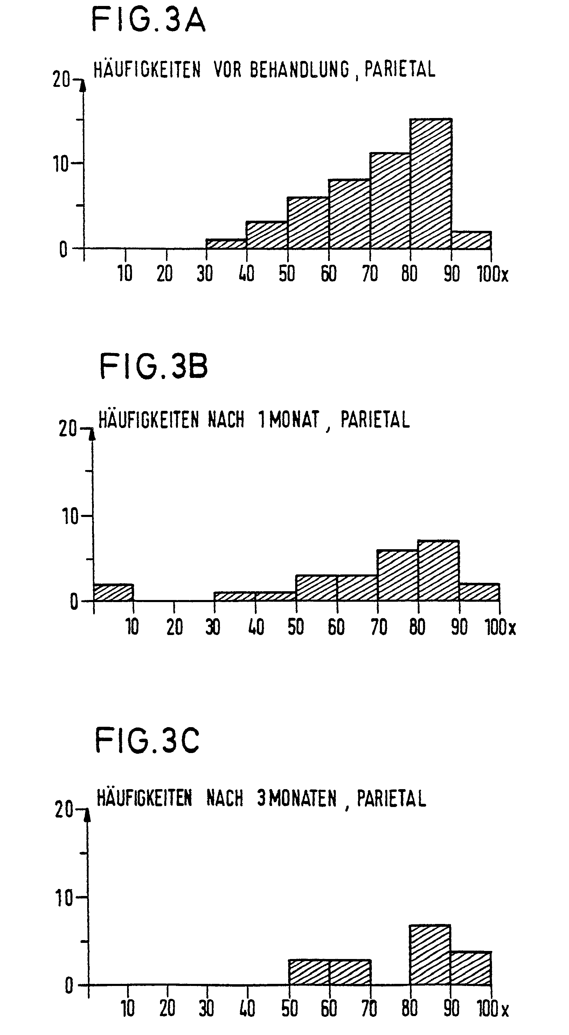

Die Figuren 1 (« Frontal »), 2 (« Occipital ») und 3 (« Parietal ») zeigen die vom Institut für Medizinische Informatik und Systemforschung der GSF Neuherberg, Prof. Dr. med. W. van Eimeren und M. Sund, M.S. nach den Untersuchungen von Prof. Braun-Falco aufgenommenen Ergebnisse der Haarwurzeluntersuchungen in der Frontal-, Parietal- und Occipitalregion des Capillitiums jeweils getrennt nach Häufigkeit vor (jeweils Fig. « A ») der Behandlung, nach einem Monat Behandlung (jeweils Fig. « B ») und nach 3 Wochen Behandlung (jeweils Fig. « C »). Hierbei wird die Prozentverteilung der anagenen Haare über die Behandlungszeit getrennt nach Beobachtungspunkten auf der Kopfhaut (frontal, parietal und occipital) aufgezeigt. Die Darstellungen zeigen nicht nur deutlich wiederum den hohen Fallanteil nahezu normaler Prozentsätze, sondern auch die hohen Fallverluste im Verlaufe der Untersuchung.

Vor Behandlung mit dem eingangs angeführten Mittel zeigten 12 Versuchspersonen starke, 6 mäßige und 4 schwache Seborrhoe des Capillitiums. Der Schweregrad der Seborrhoe, der immer von demselben Untersucher klinisch beurteilt wurde, war nach 4-wöchiger Anwendung bei 4 Versuchspersonen stark, bei 8 mäßig und bei 8 schwach. Nach 12 Wochen wurde die Seborrhoe bei je 4 Versuchspersonen schwach beurteilt. Unter den behandelten Versuchspersonen wiesen 6 starke, 6 mäßige und 2 schwache Kopfschuppung im Sinne einer Pityriasis simplex capiliti auf. Nach 4-wöchiger Behandlung fanden sich bei je 3 Versuchspersonen starke und mäßige, bei 5 Versuchspersonen schwache Schuppenbildung. Nach 12-wöchiger Behandlung fand sich in keinem Fall eine starke Kopfschuppung, bei je 5 Versuchspersonen jedoch noch mäßige und schwache Schuppenbildung.

Zur Beurteilung des Intensitätsgrades der Haarlichtung wurde die Einteilung der androgenetischen Alopezie nach Hamilton (Hamilton, J.B. : Patterned loss of hair in man : typies and incidence, Ann. of the New York Academy of Sciences 53, 708, 1951 ; Heilgemeir, G.P. : Über den Aussagewert der Haarwurzelstatusmethode, Inaug. Dissert. München 1975) zugrunde gelegt. Bei den mit dem angemeldeten Mittel behandelten Versuchspersonen fand sich 17mal der Verteilungstyp II, 13mal der Verteilungstyp IV, 4mal der Verteilungstyp V und 2mal der Verteilungstyp VI, sowie 1mal der Verteilungstyp VII. Bei 9 Versuchspersonen fand sich das typische weibliche Verteilungsmuster der androgenetischen Alopezie (female type). Über den gesamten Beobachtungszeitraum von 3 Monaten fand sich bei keiner Versuchsperson eine Änderung im Verteilungsmuster der Haarlichtung.

Bei einer Versuchsperson kam es unter der 12wöchigen Behandlung zu einer auffälligen Repigmentierung von Vellushaaren im stark gelichteten Parietalbereich. Inwieweit hier eine Repigmentierung oder eine exogene Veränderung des Haarschaftes durch das angewendete Mittel zugrunde liegt, kann aufgrund der vorliegenden Untersuchungen nicht entschieden werden.

Unter der Behandlung mit dem anspruchsgemäßen Mittel konnten keine ernstlichen Nebenwirkungen festgestellt werden. Eine Versuchsperson brach die Behandlung nach 3 Wochen wegen subjektiver Verschlechterung des Haarausfalles ab, eine weitere nach 4 Wochen wegen Brennens auf der Kopfhaut. In beiden Fällen waren bei der klinischen Kontrolluntersuchung die geklagten Beschwerden nicht objektivierbar. Ansonsten waren während des gesamten Beobachtungszeitraumes weder kosmetische störende, noch irritierende Effekte vom angemeldeten Mittel im Bereich der behaarten Kopfhaut zu beobachten.

Bei 48 Versuchspersonen mit androgenetischer Alopezie, davon 39 Männer und 9 Frauen, wurde die Wirkung einer bis zu 12-wöchigen Therapie mit dem anspruchsgemäßen Mittel auf das Haarwachstum untersucht. Dabei zeigte sich eine Besserung subjektiver Symptome wie Schmerzen im Bereich des Haarbodens und Juckreiz der behaarten Kopfhaut nach Angaben der Versuchspersonen. Ebenso ergab sich nach subjektiven Angaben der behandelten Versuchspersonen ein Nachlassen der Intensität des Effluviums, wobei betont werden kann, daß sich die Versuchspersonen fast übereinstimmend zufrieden mit der Wirkung des angemeldeten Mittels zeigten.

**Patentansprüche**

1. Haarbehandlungsmittel aus einer Lotion und einem Shampoo, dadurch gekennzeichnet, daß die Lotion eine Lösung oder Dispersion von 28 g Meerrettichsaft, 5fach konzentriert, etwa 63° Brix, 34 g Brennesselextrakt in Glykolen aufbereitet (« Extrapon »), 2 g Vitamin B6, 2 g Vitamin E wasserlöslich (1 ml = 500 mg DL-a-Tocopherolacetat), 2 g d-Panthenol, 10 g Cremophor® (Alkohol-Wasser-lösliches Rizinusöl) und 32 g Diisopropyladipat in 408 g Isopropylalkohol aufgefüllt auf 1 000 g mit destilliertem Wasser darstellt, und daß das Shampoo aus 250 g Alkyldiglykoläthersulfatnatrium, 541 g Triäthanolaminlaurylsulfat, 150 g Kokosfettdimethylaminoxyd, 8 g Meerrettichsaft, 5fach konzentriert, etwa 63° Brix, 3 g Cremophor® (Alkohol-Wasser-lösliches Rizinusöl), 9 g spanischem Rosmarinöl, 8 g Isopropylalkohol, 10 g Zitronensäure, 10 g Natriumchlorid und 20 g destilliertem Wasser besteht.

2. Haarbehandlungsmittel aus einer Lotion und einem Shampoo, dadurch gekennzeichnet, daß die Lotion eine Lösung oder Dispersion von 28 g Allylsenföl, 34 g Brennesselextrakt in Glykolen aufbereitet (« Extrapon »), 2 g Vitamin B6, 2 g Vitamin E wasserlöslich (1 ml = 500 mg DL-a-Tocopherolacetat), 2 g d-Panthenol, 10 g Cremophor® (Alkohol-Wasser-lösliches Rizinusöl) und 32 g Diisopropyladipat in 408 g Isopropylalkohol aufgefüllt auf 1 000 g mit destilliertem Wasser darstellt und daß das Shampoo aus 250 g Alkyldiglykoläthersulfatnatrium, 541 g Triäthanolaminlaurylsulfat, 150 g Kokosfettdimethylaminoxyd, 8 g Allylsenföl, 3 g Cremophor® (Alkohol-Wasser-lösliches Rizinusöl), 9 g spanischem Rosmarinöl, 8 g Isopropylalkohol, 10 g Zitronensäure, 10 g Natriumchlorid und 20 g destillierten Wasser besteht.

3. Haarbehandlungsmittel nach Anspruch 1 oder 2, dadurch gekennzeichnet, daß die Lotion 6 g sprühgetrockneten Thymusdrüsenextrakt enthält.

4. Verfahren zur Herstellung der Lotion des Haarbehandlungsmittels gemäß Anspruch 1, dadurch gekennzeichnet, daß 28 g Meerrettichsaft 5fach konzentriert, etwa 63° Brix, 35 g Brennesselextrakt in Glykolen aufbereitet (« Extrapon »), 2 g Vitamin B6, 2 g Vitamin E wasserlöslich (1 ml = 500 mg DL-a-Tocopherolacetat) 2 g d-Panthenol, 10 g Cremophor® (Alkohol-Wasser-lösliches Rizinusöl) 32 g Diisopropyladipat in 408 g Isopropylalkohol und destilliertes Wasser ad 1 000 g vermischt werden, und die entstehende Mischung nach etwa vier Wochen filtriert wird.

5. Verfahren zur Herstellung der Lotion gemäß Anspruch 2, dadurch gekennzeichnet, daß 28 g Allylsenföl, 35 g Brennesselextrakt in Glykolen aufbereitet, 2 g Vitamin B6, 2 g Vitamin E wasserlöslich (1 ml = 500 mg DL-a-Tocopherolacetat) 2 g d-Panthenol, 10 g Cremophor® (Alkohol-Wasser-lösliches Rizinusöl), 32 g Diisopropyladipat in 408 g Isopropylalkohol und destilliertes Wasser ad 1 000 g vermischt werden, und die entstehende Mischung nach etwa vier Wochen filtriert wird.

6. Verfahren zur Herstellung der Lotion nach Anspruch 3 gemäß Anspruch 4 oder 5, dadurch gekennzeichnet, daß vor der Zugabe von destilliertem Wasser ad 1 000 g 6 g sprühgetrockneter Thymusdrüsenextrakt zugegeben wird.

7. Verfahren zur Herstellung des Shampoos für das Haarbehandlungsmittel gemäß Anspruch 1 oder 3, dadurch gekennzeichnet, daß 250 g Alkyldiglykoläthersulfatnatrium, 541 g Triäthanolaminlaurylsulfat, 150 g Kokosfettdimethylaminoxid, 8 g Meerrettichsaft 5fach konzentriert, etwa 63° Brix, 3 g Cremophor® (Alkohol-Wasser-lösliches Rizinusöl), 9 g spanisches Rosmarinöl, 8 g Isopropylalkohol, 10 g Zitronensäure, 10 g Natriumchlorid und 20 g destilliertes Wasser vermischt werden.

8. Verfahren zur Herstellung des Shampoos für das Haarbehandlungsmittel gemäß Anspruch 2 oder 3, dadurch gekennzeichnet, daß 250 g Alkyldiglykoläthersulfatnatrium, 541 g Triäthanolaminlaurylsulfat, 150 g Kokosfettdimethylaminoxyd, 8 g Allylsenföl, 3 g Cremophor® (Alkohol-Wasser-lösliches Rizinusöl), 9 g spanisches Rosmarinöl, 8 g Isopropylalkohol, 10 g Zitronensäure, 10 g Natriumchlorid und 20 g destilliertes Wasser vermischt werden.

9. Anwendung des Haarbehandlungsmittels nach Anspruch 1, 2 oder 3, dadurch gekennzeichnet, daß die Kopfhaut täglich früh und abends mit der Lotion eingerieben wird und die Haare und die Kopfhaut alle zwei bis drei Tage mit dem Shampoo gewaschen werden.

## Claims

1. A hair treating agent consisting of a lotion and a shampoo, characterised in that the lotion is presented as a solution or dispersion of 28 g of horse-radish juice in five-fold concentration, about 63° Brix, 34 g stinging nettle extract prepared in glycols (« Extrapon »), 2 g vitamin B6, 2 g water-soluble vitamin E, (1 ml = 500 mg dl-α-tocopherol acetate), 2 g d-pantothenol, 10 g « Cremophor » (alcohol-water-soluble castor-oil) and 32 g diisopropyl adipate in 408 g isopropyl alcohol, made up to 1 000 g with distilled water, and the shampoo comprises 250 g sodium alkyl diglycol ether sulfate, 541 g triethanolaminolauryl sulfate, 150 g coconut fat-dimethylamino oxide, 8 g horse-radish juice in five-fold concentration, about 63° Brix, 3 g « Cremophor » (alcohol-water-soluble castor oil), 9 g Spanish rosemary oil, 8 g isopropyl alcohol, 10 g citric acid, 10 g sodium chloride and 20 g distilled water.

2. A hair treating agent consisting of a lotion and a shampoo characterised in that the lotion is presented as a solution or dispersion of 28 g allyl mustard oil, 34 g stinging nettle extract prepared in glycols (« Extrapon »), 2 g vitamin B6, 2 g water-soluble vitamin E (1 ml = 500 mg dl-α-tocopherol acetate), 2 g d-pantothenol, 10 g « Cremophor » (alcohol-water-soluble castor oil) and 32 g diisopropyl adipate in 408 g isopropyl alcohol made up to 1 000 g with distilled water and the shampoo comprises 250 g sodium alkyl diglocol ether sulfate, 541 g triethanolaminolauryl sulfate, 150 g coconut fat-dimethylamineoxide, 8 g allyl mustard oil, 3 g « Cremophor » (water-alcohol-soluble castor oil), 9 g Spanish rosemary oil, 8 g isopropyl alcohol, 10 g citric acid, 10 g sodium chloride and 20 g distilled water.

10

3. The hair treating agent according to Claim 1 or 2, characterised in that the lotion contains 6 g spraydried thymus gland extract.

4. A method of preparing a lotion of the hair treating agent according to Claim 1 characterised in that 28 g of horse-radish juice, in five-fold concentration, about 63° Brix, 35 g of stinging nettle extract prepared in glycols, (« Extrapon »), 2 g of vitamin B6, 2 g of water-soluble vitamin E (1 ml = 500 mg of dl-α-tocopherol acetate), 2 g of d-pantothenol, 10 g of « Cremophor » (alcohol-water-soluble castor oil), 32 g of diisopropyl adipate in 408 g of isopropyl alcohol and distilled water to make up 1 000 g are mixed, and the resulting mixture is filtered after about four weeks.

5. A method of preparing the lotion according to Claim 2 characterised in that 28 g of allyl mustard oil, 35 g of stinging nettle extract prepared in glycols, (« Extrapon »), 2 g of vitamin B6, 2 g of water-soluble vitamin E (1 ml = 500 mg of dl-α-tocopherol acetate), 2 g of d-pantothenol, 10 g of « Cremophor » (alcohol-water-soluble castor oil), 32 g of diisopropyl adipate in 408 g of isopropyl alcohol and distilled water to make up 1 000 g are mixed and the resulting mixture is filtered after about four weeks.

6. A method of preparing the lotion of Claim 3 according to Claim 4 or 5, characterised in that before the addition of distilled water to make up 1 000 g, 6 g spray-dried thymus gland extract are added.

7. A method of preparing the shampoo for the hair treating agent according to Claim 1 or 3, characterised in that 250 g of sodium alkyl diglycol ether sulfate, 541 g of tricethanolaminolauryl sulfate, 150 g of coconut fat-dimethylamineoxide, 8 g of horse-radish juice in five-fold concentration, about 63° Brix, 3 g of « Cremophor » (alcohol-water-soluble cator oil), 9 g of Spanish rosemary oil, 8 g of isopropyl alcohol, 10 g of citric acid, 10 g of sodium chloride and 20 g of distilled water are mixed.

8. A method of preparing the shampoo for the hair treating agent according to Claims 2 or 3, characterised in that 250 g of sodium alkyl diglycol ether sulfate, 541 g of triethanolaminolauryl sulfate, 150 g of coconut fat-dimethylamineoxide, 8 g allyl mustard oil, 3 g of « Cremophor » (alcohol-water-soluble castor oil), 9 g of Spanish rosemary oil, 8 g of isopropyl alcohol, 10 g of citric acid, 10 g of sodium chloride, 20 g of distilled water are mixed.

9. The use of the hair treating agent according to Claim 1, 2 or 3, characterised in that the lotion is rubbed into the skin of the head daily, in the morning and in the evening, and the hair and the skin of the head are washed with the shampoo every two to three days.

## Revendications

1. Remède pour le traitement des cheveux, constitué d'une lotion et d'un shampooing, caractérisé en ce que la lotion comporte une solution ou dispersion de 28 g de jus de raifort en quintuple concentration, environ 63° brix, 34 g d'extrait d'ortie préparé en glycols (« Extrapon »), 2 g de vitamine B6, 2 g de vitamine E soluble dans l'eau (1 ml = 500 mg DL-a-tocophérolacétate), 2 g de d-panthénol, 10 g de Cremophor® (huile de ricin soluble dans l'eau — alcool) et 32 g de diisopropyladipate dans 408 g d'alcool isopropylique additionnés d'eau distillée de manière à obtenir 1 000 g, et en ce que le shampooing se compose de 250 g de sodium alkyldiglycoléthersulfate, 541 g de laurylsulfate triéthanolamine, 150 g de diméthylaminoxyde de coprah, 8 g de jus de raifort en quintuple concentration, environ 63° brix, 3 g de Cremophor® (huile de ricin soluble dans l'eau-alcool), 9 g d'essence de romarin espagnol, 8 g d'alcool isopropylique, 10 g d'acide citrique, 10 g de chlorure de sodium et 20 g d'eau distillée.

2. Remède pour le traitement des cheveux, constitué d'une lotion et d'un shampooing, caractérisé en ce que la lotion comporte une solution ou dispersion de 28 g d'huile allylique de moutarde, 34 g d'extrait d'ortie préparé en glycols (« Extrapon »), 2 g de vitamine B6, 2 g de vitamine E soluble dans l'eau (1 ml = 500 mg DL-a-tocophérolacétate), 2 g de d-panthénol, 10 g de Cremophor® (huile de ricin soluble dans l'eau-alcool) et 32 g de diisopropyladipate dans 408 g d'alcool isopropylique additionnés d'eau distillée de manière à obtenir 1 000 g, et en ce que le shampooing se compose de 250 g de sodium alkyldiglycoléthersulfate, 541 g de laurylsulfate triéthanolamine, 150 g de diméthylaminoxyde de coprah, 8 g d'huile allylique de moutarde, 3 g de Cremophor® (huile de ricin soluble dans l'eau-alcool), 9 g d'essence de romarin espagnol, 8 g d'alcool isopropylique, 10 g d'acide citrique, 10 g de chlorure de sodium et 20 g d'eau distillée.

3. Remède pour le traitement des cheveux suivant la revendication 1 ou 2, caractérisé en ce que la lotion contient 6 g d'extrait de thymus séché par pulvérisation.

4. Procédé pour la production de la lotion du remède pour le traitement des cheveux suivant la revendication 1, caractérisé en ce qu'il est mélangé 28 g de jus de raifort en quintuple concentration, environ 63° brix, 35 g d'extrait d'ortie préparé en glycols (« Extrapon »), 2 g de vitamine B6, 2 g de vitamine E soluble dans l'eau (1 ml = 500 mg DL-a-tocophérolacétate), 2 g de d-panthénol, 10 g de Cremophor® (huile de ricin soluble dans l'eau-alcool), 32 g de diisopropyladipate dans 408 g d'alcool isopropylique et de l'eau distillée pour obtenir 1 000 g, et en ce que le mélange obtenu est filtré après environ quatre semaines.

5. Procédé pour la production de la lotion suivant la revendication 2, caractérisé en ce qu'il est mélangé 28 g d'huile allylique de moutarde, 35 g d'extrait d'ortie préparé en glycols, 2 g de vitamine B6, 2 g de vitamine E soluble dans l'eau (1 ml = 500 g DL-a-tocophérolacétate), 2 g de d-panthénol, 10 g de Cremophor® (huile de ricin soluble dans l'eau-alcool), 32 g de diisopropyladipate dans 408 g d'alcool isopropylique et de l'eau distillée pour obtenir 1 000 g, et ce que le mélange obtenu est filtré après environ quatre semaines.

6. Procédé pour la production de la lotion suivant la revendication 3 et suivant la revendication 4 ou 5, caractérisé en ce qu'il est ajouté 6 g d'extrait de thymus séché par pulvérisation, ayant adjonction d'eau distillée de manière à obtenir 1 000 g.

7. Procédé pour la production du shampooing destiné au remède pour le traitement des cheveux suivant la revendication 1 ou 3, caractérisé en ce qu'il est mélangé 250 g de sodium alkyldiglycoléthersulfate, 541 g de laurylsulfate triéthanolamine, 150 g de diméthylaminoxyde de coprah, 8 g de jus de raifort en quintuple concentration, environ 63° brix, 3 g de Cremophor® (huile de ricin soluble dans l'eau-alcool), 9 g d'essence de romarin espagnol, 8 g d'alcool isopropylique, 10 g d'acide citrique, 10 g de chlorure de sodium et 20 g d'eau distillée.

8. Procédé pour la production du shampooing destiné au remède pour le traitement des cheveux suivant la revendication 2 ou 3, caractérisé en ce qu'il est mélangé 250 g de sodium alkyldiglycoléthersulfate, 541 g de laurylsulfate triéthanolamine, 150 g de diméthylaminoxyde de coprah, 8 g d'huile allylique de moutarde, 3 g de Cremophor® (huile de ricin soluble dans l'eau-alcool), 9 g d'essence de romarin espagnol, 8 g d'alcool isopropylique, 10 g d'acide citrique, 10 g de chlorure de sodium et 20 g d'eau distillée.

9. Emploi du remède pour le traitement des cheveux suivant la revendication 1, 2 ou 3, caractérisé en ce que le cuir chevelu est frictionné quotidiennement, le matin et le soir, avec la lotion et en ce que les cheveux et le cuir chevelu sont lavés tous les deux à trois jours avec le shampooing.

## FIG.1A

HÄUFIGKEITEN VOR BEHANDLUNG, FRONTAL

## FIG.1B

HÄUFIGKEITEN NACH 1 MONAT, FRONTAL

## FIG.1C

HÄUFIGKEITEN NACH 3 MONATEN, FRONTAL

# FIG.2A

HÄUFIGKEITEN VOR BEHANDLUNG, OCCIPITAL

# FIG.2B

HÄUFIGKEITEN NACH 1MONAT, OCCIPITAL

# FIG.2C

HÄUFIGKEITEN NACH 3 MONATEN, OCCIPITAL

# FIG.3A

HÄUFIGKEITEN VOR BEHANDLUNG , PARIETAL

# FIG.3B

HÄUFIGKEITEN NACH 1 MONAT , PARIETAL

# FIG.3C

HÄUFIGKEITEN NACH 3 MONATEN , PARIETAL